# EUROPEAN PATENT APPLICATION

(11) **EP 3 648 293 A1**
(43) Date of publication of application: **06.05.2020**
(21) Application number: 18204225.9
(22) Date of filing: 03.11.2018
(51) Int. Cl.: H02J 7/02, H01M 10/46, A61B 5/00, H02J 50/10, H02J 7/00

(54) **BATTERY HOUSING**

(71) Applicant: Indigo Diabetes N.V., 9052 Gent (BE)
(72) Inventor: VAN SCHUYLENBERGH, Koenraad, 2290 Vorselaar (BE); ORDONEZ ORELLANA, Juan Sebastian, 9000 Gent (BE)
(74) Representative: DenK iP

(57) **Abstract**

A battery housing (100) for use in an active implantable medical device comprising a battery (20) and a signal transferring element (30) is described. The battery housing (100) is adapted for holding the signal transferring element at a fixed position around the battery housing (100). A corresponding implantable device (10) also is disclosed.

## Description

### Field of the invention

The invention relates to the field of implantable devices. More specifically it relates to implantable devices comprising a battery as well as an inductive link for providing data and/or power signal transferring between the implanted device and an external unit.

### Background of the invention

In general, there are multiple reasons for making implantable devices as small as possible: smaller devices are less invasive, provide a better body acceptance, are more compliant with movement, are more easy to implant since there is less restriction on where they can be implanted, etc. However, a small device size also restricts the amount of energy that can be stored in an on-board battery. Batteries in small implantable devices therefore require frequent recharge, typically wirelessly through the skin over an inductive link. The induction coil inside the implantable device should be made as large as possible within the device constraints, to maximize the energy efficiency of the inductive link, and thereby minimize the recharge time and heat dissipated in the device. The larger the area enclosed by the implanted-device coil, the higher its magnetic coupling with the external-unit coil and the better the energy efficiency of the wireless link.

Also, communication elements like coils and antennas require precise dimensioning, wire placement, and positioning with respect nearby conductive structures like a battery, to produce specific inductances (L), resonance and loss characteristics. It is common practice to form the coil on a coil-former that provides a body to wrap the coil around and enables precise mechanical dimensions. Then, the individual coil windings are glued to each other to maintain the relative distance to each other and form a mechanically strong structure. Sometimes, coils can also be potted in resin, epoxy, silicone rubber, etc to form the solid structure and lock the windings in place. For free-standing coils without permanent former, a temporary coil-forming feature (e.g. a rod) is made of a non-sticky material (e.g. Teflon) and is removed after glueing/molding the coil wires together to create a mechanically strong assembly.

Others implement printed coils, which are integrated into the printed circuit boards. This coil configuration is however not well suited for all frequencies and communication coupling requirements.

### Summary of the invention

It is an object of embodiments of the present invention to provide good implantable devices comprising a battery and a signal transferring element, such as for example a data signal and/or power signal transferring element. It is an advantage of embodiments of the present invention that battery and signal transferring element are designed and positioned such that good, e.g. optimum, use of the available space is provided, while maintaining appropriate functioning of both the signal transferring element and the battery.

It is an advantage of embodiments of the present invention that the use of volume within a limited-space environment in an implantable device is optimized by providing a battery designed and machined to contain one or more structural features (beyond encasing the battery's chemistry) to provide guidance, accommodation and/or fixation of the signal transferring element, e.g. a power signal or data signal communication element, such as for example an inductive link. It is an advantage of embodiments of the present invention that these structural features can be micrometer-precise, so that the guidance, accommodation and fixation can be very accurate.

It is an advantage of embodiments of the present invention that one or more capacitors used in the signal transferring element, e.g. inductive link, can be provided in a dedicated space, so that the signal transferring elements can be assembled and tested in a reproducible manner in a subassembly prior to installation into the implantable device. This ensures that the dimensions and/or absolute position in the implantable device can be optimized while giving maximum use of the available space.

It is an advantage of embodiments according to the present invention that the signal transferring element and the battery are not placed distinctively from each other but that their position is optimized so as to make optimum use of the available place in the implantable device.

It is an advantage of embodiments of the present invention that the battery body is formed to maximize space use for the battery and at the same time provide the coil-forming structure.

It is an advantage of at least some embodiments of the present invention that structures (spacers/feet) are provided by the battery to mount subcomponents elevated over other components. Such structures, e.g. spacers, allow allocation of other parts with different heights at the required locations underneath the body of the battery. This provides optimal volume use for all components.

It is an advantage of embodiments of the present invention that by shaping the body of the battery (e.g. machining or molding) to contain mechanical features of help for coil forming, it is possible to produce extremely precise coils, reducing dimensional variations in shape and position from product to product. It is an advantage of embodiments of the present invention that tolerances can be controlled in a better way. It is furthermore an advantage of embodiments of the present invention that the shape of the coil can be maintained as it was formed, so it does not have to be removed from the coil former and used as a delicate component.

It is an advantage of embodiments of the present invention that by using the battery housing as the positioning element of the signal transferring element, e.g. as the coil former, a higher reproducibility of the signal transferring properties can be obtained, such as for example a higher reproducibility of the coil inductance value can be obtained. Since a battery contains metals and other highly conductive materials inside, a coil of the signal transferring element, may induce eddy currents in those materials. These eddy currents reduce the effective coil inductance and increase the magnetic losses. The closer the battery is positioned to the coil the more pronounced the effect; the lower the effective coil inductance and quality factor. Winding the signal transferring element or elements thereof around the battery housing, or forming a flexible substrate with the signal transferring elements around the battery housing, or introducing the signal transferring element directly on the battery housing, creates a highly reproducible relation between the battery and the signal transferring element. As a result, the eddy current effect is reproducible and consistent between different signal transferring elements.

The present invention relates to a battery housing for use in an active implantable medical device comprising a battery and a signal transferring element, the battery housing being adapted for positioning the signal transferring element at a fixed position around the battery housing. It is an advantage of embodiments of the present invention that an accurate position of the coil with respect to the battery can be determined.

The battery housing may comprise sidewalls adapted in shape for allowing positioning of the signal transferring element around the sidewalls. It is an advantage of embodiments of the present invention that delimitation of the position of the coil can be accurately obtained. The battery housing may comprise an outstanding protrusion on the sidewalls for positioning the signal transferring element. It is an advantage of embodiments of the present invention that sliding off of the coil can be avoided.

The battery housing may comprise reinforcement ribs for reinforcing the sidewalls. It is an advantage of embodiments of the present invention that mechanical deformation due to forces acting on the thin, coil-forming walls by the coil can be limited or even avoided.

The battery housing may be adapted for positioning the signal transferring element around the battery housing by one or more of
- the battery housing being adapted for positioning a flexible substrate comprising the signal transferring element around the battery housing,
- the battery housing having the signal transferring element integrated on or in the battery housing or
- the battery housing being adapted for holding a coil, for a signal transferring element having a coil, at a fixed position around the battery housing.

The signal transferring element may be a capacitive or inductive signal transferring element or an antenna.

The battery housing may comprise an extension comprising an upstanding sidewall for extending the dimensions of the coil relative to the possible battery volume. It is an advantage of embodiments of the present invention that the coil enclosed surface can be larger than the surface defined by the battery.

The battery housing may comprise a compartment for accommodating tall components. The compartment may be a recess in the battery housing. The recess may be open from the upper side of the battery housing or may be closed from the upper side of the battery housing.

The battery housing may comprise guiding delimitations and notches to precisely position the wire for connecting the signal transferring element to another component without deforming the signal transferring element.

The battery housing may comprise fixation features to guide and fixate a wire for connecting the signal transferring element to another component at an interconnection position. The fixation features may be snapping features for fixing a wire of the coil.

The battery housing may comprise space and recesses to place the capacitors to create the resonant tank circuit for an inductive link and recesses and holding elements to accommodate metal contacts to create an electrical connection between the capacitors and the signal transferring element.

The battery housing may comprise a recess to accommodate shallow components.

The present invention also relates to an active implantable medical device comprising a battery and a signal transferring element, the active implantable medical device furthermore comprising a battery housing as described above for fitting the battery.

The device may comprise any of spring contacts, wired contacts, jacks as plug-in terminals or plugs as plug-in terminals, for contacting to the battery or to the coil and/or wherein the device comprises a flexible circuit board extending the battery/coil contacts towards an interconnection. The contacts may have any shape or be of any kind for providing a compliant interconnection to the battery or the coil. The active implantable medical device may be an electronic implantable device.

The interconnection may be may through a ZIF connector, or any other suitable connector. The active implantable medical device may be a sensor for sensing any of glucose, urea, creatinine, triglyceride, protein, cholesterol, ethanol, ketones, hormones or lactate.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG 1, FIG. 2 and FIG. 3 are battery housings according to embodiments of the present invention.
FIG. 4 illustrates a schematic overview of the battery housing part of an active implantable medical device according to an embodiment of the present invention.
FIG. 5 and FIG. 6 illustrate a battery housing for an active implantable medical device according to an alternative embodiment of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

Where in embodiments of the present invention reference is made to a signal transferring element, reference may be made to a communication element for communicating one or more data signals from and/or to the implantable device. Alternatively or in addition thereto, the signal transferring element also may refer to a power signal transferring element, which may also be referred to as power communicating element, for powering one or more components, e.g. for powering a battery of the implantable device. The signal transferring element may be based on inductive operation as well as capacitive operation, resistive operation or electromagnetic radiation. Signal transferring elements based on inductive operation typically may comprise a coil. Signal transferring elements based on electromagnetic radiation typically may comprise an antenna.

In a first aspect, a battery housing is provided, which defines and fixes the position of a part or all of a signal transferring element, specifically the element for transferring electromagnetic signals, e.g. which defines and fixes the position and configuration of a coil of a signal transferring element, or in general of an antenna of a signal transferring element. The latter can be obtained in a plurality of ways. In some embodiments, this may be obtained by a component, e.g. a coil, of the signal transferring element being wound around the battery housing. In other embodiments this may be obtained by the battery housing being adapted for winding a flexible substrate with the signal transferring element integrated therein or thereon around the housing. In yet other embodiments, the signal transferring element may be directly integrated in or on the battery housing.

The battery housing may include features for positioning these elements in a precise and consistent manner. The battery housing can be assembled as a subassembly, including other elements such as metallic contacts and the element for coupling electromagnetic signals, and it can be tested before installation into a system. The accurate and repeatable positioning allows determining in an accurate way the influences (e.g. inductive influences) of the signal transferring element over other features, structures and subsystems of the device, e.g. over metallic parts.

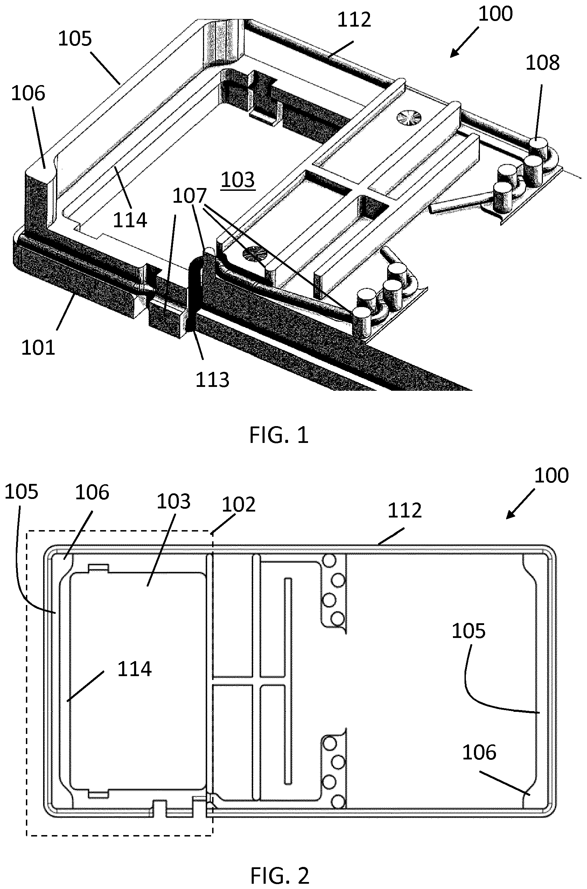

FIG. 1, FIG. 2 and FIG. 3 show the perspective, top view and lateral view respectively of a battery housing 100 according to some embodiments of the present invention, for an implantable device. The battery housing 100 is adapted to hold a component of the signal transferring element, in the present example being a coil 112, 113 of a signal transferring element, e.g. as a part of an inductive link. For example, the design and shape of the body may provide any or all of accommodation, guidance and fixation of the component, e.g. of a wire of the coil 112, 113. Thus, the component position with respect to the battery can be determined in a consistent and repeatable way.

The battery housing 100 may include mechanical features to hold and accommodate and/or fixate the component 112, 113 to it.

In some embodiments of the present invention, the battery housing 100 itself is used as a coil-forming structure. The battery housing 100 may include mechanical features which facilitates coil forming, with no need to remove the coil once formed. These mechanical features may be the same as the features to hold the coil 112, 113.

In some embodiments, the battery housing 100 includes sidewalls 105 with a shape such that winding of the coil 112, 113 can be provided. This allows an accurate delimitation of the positioning of the coil 112, 113. FIG. 2 clearly shows the sidewalls 105 at opposite sides of the battery housing 100, thus allowing a coil wire to wind around the sidewalls 105, thereby enclosing a surface including the battery.

Back to FIG. 1, the exemplary battery housing 100 includes at least one outstanding protrusion 101 on the sidewalls 105, which prevents the wire of the coil 112, 113 from sliding off the battery housing 100. FIG. 1 shows the protrusion 101 at the bottom of the sidewall 105, but it may be on the top, or more than one protrusions (e.g. one at the top and one at the bottom of the sidewall) may be provided.

The battery housing 100 may include reinforcement ribs 106 as shown in FIG. 1 and top view of FIG. 2. The ribs 106 reduce or prevent bending or deformation of the sidewalls 105. The sidewalls 105 can be made thin thus reducing materials and weight, and still being reinforced by the ribs, reducing mechanical deformations due to forces acting on the coil-forming walls. The ribs 106 may be included as part of the sidewalls 105, and they may be distributed along the sidewalls 105. For example, at least two may be included, e.g. at the lateral borders of the sidewall 105 as shown in the figures.

In some embodiments, the type of transmission to be performed with the device may require large elements, such as a winding enclosing a large area. This may not be compatible with the shape and/or size of the battery, which is usually limited by capacity and power requirements, and it is preferable small. The size and shape (e.g. the perimeter) of the battery housing 100 may be extended to meet coiling requirements. In some embodiments of the present invention, the battery housing 100 as shown in its top view (FIG. 2) includes an extension 102 (indicated with dashed lines). This extension 102 serves to allow extending the dimensions of the coil 112, 113 relative to the volume of a battery included in the battery housing 100. The extension may include at least one of the sidewalls 105 of the battery housing 100.

In some embodiments of the present invention, the battery housing 100 comprises a compartment 103 for accommodating additional components, for example components of the battery and/or components of the implantable device, e.g. electronic components of the implantable device. For example, the compartment may be used to accommodate tall components. For example, the compartment may be used to stack additional components. A cavity protrusion 114 may be included to hold additional components.

The cavity 103 may be open or not, for example it may be opened or closed from one of the sides of the battery housing, for example from the upper side of the battery housing. An open cavity may provide easy installation of additional tall components.

In some embodiments of the present invention, the compartment 103 may be provided within the extension 102, thus combining the extension of coil winding with the compartment 103 for additional components for providing a compact battery housing 100.

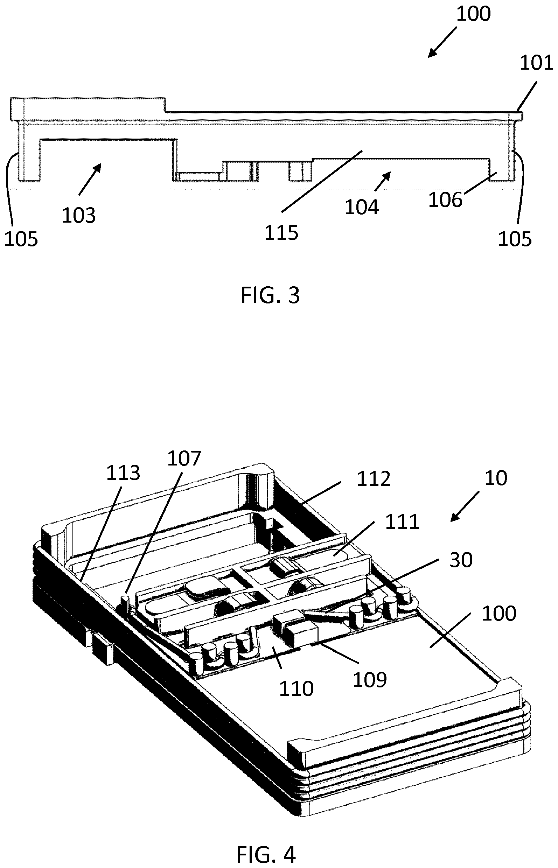

As shown in FIG. 3, the sidewalls 105 of two opposite sides of the battery housing 100 (e.g. the sides along the width) may be taller than the walls 115 of the other two opposite sides of the battery housing 100 (e.g. the sides along the length), saving material and potentially improving positioning of the coil 112, 113. In some embodiments, the tall sidewalls 105 may surround the battery housing 100, and/or they may include portions of different shapes and/or heights.

In some embodiments of the present invention, the battery housing 100 may include guiding delimitations and notches 107 such as the ones shown in FIG. 1, for precisely positioning the wire, for example holding it, without deforming the wire, for example without deforming the cross section of the wire of (or connected to) a coil. This may allow reducing mechanical stresses and obtaining a resistance substantially consistent along the wire. The guiding delimitations and notches 107 may be provided for example on the protrusion 101, and/or on the extension 102 of the housing, although the present invention is not limited thereto, and they may be provided in portions of the walls 215 of the battery housing 100, and/or on the sidewall 105.

For example, in some embodiments, the corners at the borders of the sidewall 105 may be rounded, reducing stresses on the wire of the coil 112, 113, and allowing winding without deforming the coil.

The guiding delimitations and notches 107 may allow providing part of a conducting wire underneath the rest of the coil, sandwiched between the coil 113 and the housing, and/or embedding portions of said wire within the battery housing 100, e.g. parts of the battery housing 100 may protect portions of the wire of the coil, while guiding them towards the contacts of the coil which may be provided in the battery housing 100.

The battery housing may include fixation features 108 for fixing a wire of the coil (e.g. a wire for connecting the coil to other elements), as indicated in the exemplary embodiment of FIG. 1. For example, snapping features can be provided, or protrusions such as clamping protrusions can be provided, for example tabs or the like, which may hold a portion of conducting wire in zig-zag. Other fixation features may be included, such as adhesive, loops provided on the battery housing, and the like.

The battery housing may further include space and recesses to place further components of an implantable device, e.g. a signal transferring element thereof. The battery housing 100 shown in FIG. 4 includes space and recesses 109 provided in the battery housing 100. Said space and recesses 109 may include for example capacitors to create a resonant tank circuit for the inductive link of a signal transferring element including the coil 112, 113.

In some embodiments of the present invention, the housing may further include recesses and holding elements 110 to accommodate metal contacts 111, for providing an electrical connection between capacitors and coil.

Additionally, further components may be included to the battery housing, for example shallow components. A recess 104 for shallow components can be provided in order to integrate these shallow components, while tall components can be fitted in the cavity 103 (which may also or alternatively fit shallow components, which may be e.g. stacked). An exemplary recess 104 for shallow components is clearly illustrated in FIG. 3, delimited by one of the sidewalls 105.

The battery housing 100 may comprise or made of a material suitable for housing a battery. The battery housing 100 may be made of a single piece, for example by moulding or machining.

The present invention is not limited to battery housings adapted to hold coils of signal transferring devices. In other embodiments, an antenna can be provided, for example a planar antenna or the like. More generally a component, e.g. coil or antenna, of a signal transferring element may be integrated on or in a flexible substrate that can be wrapped around or positioned fixed to the battery housing or walls thereof.

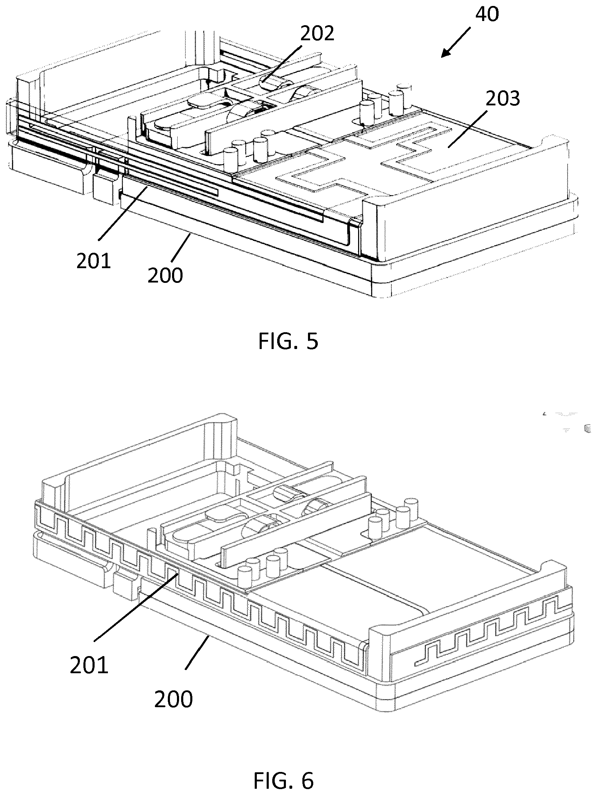

FIG. 5 illustrates an exemplary embodiment of the battery housing 200 according to embodiments of the present invention, adapted to hold an antenna 201 of a signal transferring element. The antenna 201 may be provided on a wall of the housing, and/or on the sidewall, for example it may extend around the perimeter of the battery housing 200. The antenna 201 may comprise a conductive layer, for example a shaped metallic layer fixed to the battery housing 200. The layer may be provided by shaping a sheet, or by other means such as printing, plating, or patterning.

In some embodiments of the present invention, a substrate 202 may be provided, which may be flexible. It may be provided at least on a portion of the perimeter of the battery housing 200; for example it may wrap around the battery housing 200, fixing the position of the antenna 201. In the particular example shown in FIG. 5 the antenna 201 is embedded within the flexible substrate 202 which provides good positioning and fixation. However, the present invention is not limited to this example, and the antenna 201 may be sandwiched between the side walls 215 of the battery housing and the substrate 202, which is easy to provide. For example, the antenna 201 may be positioned on a flexible substrate 202, for example adhered thereon, or printed thereon.

FIG. 5 also shows conductive tracks 203 directly provided on the battery housing 200, for example on its surface, for example on the recess 204 for shallow components, for electrically connecting (e.g. by direct ohmic connection) these devices and element to the antenna 201.

FIG. 6 illustrates a similar device as shown in FIG. 5 whereby a different antenna 201 shape is shown for a battery housing 200.

In yet other embodiments, the signal transferring element may be integrated in or on the battery housing. The latter can be performed in any suitable way, such as for example by inscribing the signal transferring element, by imprinting, by 3D printing, by plating, by patterning, or in any other possible way.

In a second aspect, an implantable device is provided, comprising a battery housing as described in the first aspect.

As indicated above, FIG. 4 illustrates a schematic overview of a battery housing part of an active implantable medical device 10, in according with an embodiment of the present invention. Such an active implantable medical device includes a battery housing 100 in accordance with embodiments of embodiments of the previous aspect. The features of the battery housing 100 explained with reference to FIG. 1, FIG. 2, FIG. 3 and FIG. 4 are applicable to the implantable device. In particular, it shows the routing of the wire thanks to the guiding limitations and notches 107, according to an example of an embodiment of the present invention, allowing the conducting wire to extend underneath the rest of the coil, sandwiched between the coil 113 and the housing. It further includes a signal transferring device 30 including the coil.

FIG. 5 illustrates a schematic overview of an alternative active implantable medical device 40 including a battery housing 200, which may for example include some or all of the features of the previous aspect, but a different electromagnetic coupling element, in this case the antenna 201 mentioned earlier. The use of an antenna may require different positioning of some elements. Some elements with specific use for wires, such as some delimitations and notches 107 specifically adapted for routing wires, may be not provided thus allowing reduction of costs, as this specific embodiment does not use wires or coils.

The antenna 201 and/or the battery can be connected to further devices and elements of an implantable device in any suitable way. For example, it can be connected to further devices by providing contacts electrically coupled to the conductive tracks 203.

For example, electrical connection to the battery (or to the coil or the antenna, or in general to the signal transferring element 30) may be provided by spring contacts, wired contacts, jacks as plug-in terminals or plugs as plug-in terminals. In some embodiments of the present invention, the implantable device may include a flexible circuit board extending the battery and/or coil contacts towards an interconnection.

The device can be used as a sensor for sensing any of glucose, urea, creatinine, triglyceride, protein, cholesterol, ethanol, ketones, hormones or lactate. A highly integrated and compact implantable device, providing data communication and/or wireless charging of the battery, can be provided in a reliable way.

## Claims

1. A battery housing (100) for use in an active implantable medical device (10) comprising a battery (20) and a signal transferring element (30), the battery housing (100) being adapted for positioning the signal transferring element (30) at a fixed position around the battery housing (100).

2. A battery housing (100) according to claim 1, wherein the battery housing (100) comprises sidewalls (105) adapted in shape for allowing positioning of the signal transferring element around the sidewalls (105).

3. A battery housing (100) according to claim 2, wherein the battery housing (100) comprises an outstanding protrusion (101) on the sidewalls for positioning the signal transferring element (112, 113).

4. A battery housing (100) according to claims 2 or 3, wherein the battery housing (100) comprises reinforcement ribs (106) for reinforcing the sidewalls (105).

5. A battery housing (100) according to any of the previous claims, wherein battery housing (100) is adapted for positioning the signal transferring element around the battery housing by one or more of
- the battery housing being adapted for positioning a flexible substrate comprising the signal transferring element (30) around the battery housing,
- the battery housing having the signal transferring element (30) integrated on or in the battery housing or
- the battery housing being adapted for holding a coil, for a signal transferring element (30) having a coil, at a fixed position around the battery housing (100).

6. A battery housing (100) according to any of the previous claims, wherein the signal transferring element is a capacitive or inductive signal transferring element or an antenna.

7. A battery housing (100) according to any of the previous claims, wherein the battery housing (100) comprises an extension (102) comprising an upstanding sidewall (105) for extending the dimensions of the coil (112, 113) relative to the possible battery volume.

8. A battery housing (100) according to any of the previous claims, wherein the battery housing comprises a compartment (103) for accommodating tall components.

9. A battery housing (100) according to any of the previous claims, the battery housing (100) comprising guiding delimitations and notches (107) to precisely position the wire for connecting the signal transferring element to another component without deforming the signal transferring element (112, 113).

10. A battery housing (100) according to any of the previous claims, the battery housing (100) comprising fixation features (108) to guide and fixate a wire for connecting the signal transferring element to another component at an interconnection position.

11. A battery housing (100) according to any of the previous claims, the battery housing (100) comprising space and recesses (109) to place the capacitors to create the resonant tank circuit for an inductive link and recesses and holding elements (110) to accommodate metal contacts to create an electrical connection between the capacitors and the signal transferring element.

12. A battery housing (100) according to any of the previous claims, the battery housing (100) comprising a recess (104) to accommodate shallow components.

13. An active implantable medical device (10) comprising a battery (20) and a signal transferring element(30), the active implantable medical device furthermore comprising a battery housing (100) according to any of claims 1 to 10 for fitting the battery (20).

14. An active implantable medical device (10) according to claim 11, wherein the device comprises any of spring contacts, wired contacts, jacks as plug-in terminals or plugs as plug-in terminals, for contacting to the battery or to the coil and/or wherein the device comprises a flexible circuit board extending the battery/coil contacts towards an interconnection.

15. An active implantable medical device (10) according to any of claims 11 to 13, wherein the active implantable medical device (10) is a sensor for sensing any of glucose, urea, creatinine, triglyceride, protein, cholesterol, ethanol, ketones, hormones or lactate.
